# EUROPEAN PATENT APPLICATION

(11) **EP 4 311 532 A1**
(43) Date of publication of application: **31.01.2024**
(21) Application number: 22187371.4
(22) Date of filing: 28.07.2022
(51) Int. Cl.: A61G 13/10, A61B 6/00

(54) **MEDICAL SETUP, SYSTEM FOR CALIBRATING AN OPERATING TABLE IN A MEDICAL ENVIRONMENT, MOBILE DEVICE AND METHOD FOR OPERATING MEDICAL EQUIPMENT**

(71) Applicant: Koninklijke Philips N.V., 5656 AG Eindhoven (NL)
(72) Inventor: TRIVEDI, Vithal Virendra, Eindhoven (NL)
(74) Representative: Philips Intellectual Property & Standards

(57) **Abstract**

The invention relates to a medical setup (9), comprising a mobile device (1) and a medical system (10). The mobile device (1) comprises at least one position sensor (2), a wireless communication unit (4) and mounting elements (8) for attaching the mobile device (1) to a defined location (20) of an operating table (11). The medical system (10) comprises a wireless communication base unit, a computing unit and a reference location (16) arranged to mount the mobile device (1). The computing unit is configured to calibrate an operating table (11) with respect to the medical system, store the defined location as a known location in a coordinate system of the medical system and receive position information related to the measurements from the at least one position sensor (2) via the wireless communication base unit. The invention further relates to a system for calibrating an operating table (11), to a mobile device (1) and to a method for operating medical equipment.

## Description

### FIELD OF THE INVENTION

The invention relates to a medical setup, to a system for calibrating an operating table in a medical environment, to a mobile device and to a method for operating medical equipment.

### BACKGROUND OF THE INVENTION

Mobile surgery systems, such as mobile C-arm X-ray systems, are often sold without a dedicated operating table and hence allow a combination with a plurality of operating tables, often from different manufacturers. This leads to an unknown position of the operating table and/or of a patient lying on the operating table.

However, for many applications, e.g., when the mobile surgery system is motorized and/or when the mobile surgery system is used as a catherization laboratory, there is a need to know the position of the patient and/or operating table with respect to the mobile surgery system.

### SUMMARY OF THE INVENTION

It is therefore an object of the present invention to provide a medical setup, a system for calibrating an operating table in a medical environment, a mobile device and a method for operating medical equipment that calibrate the relative position of the operating table.

The object of the present invention is solved by the subject matter of the independent claims, wherein further embodiments are incorporated in the dependent claims.

In an aspect of the present invention, a medical setup is provided. The medical setup comprises a mobile device and a medical system.

The mobile device is, in particular, a hand-held device. It comprises at least one position sensor and a wireless communication unit. The at least one position sensor is adapted to determine a position, in particular a relative position, of the mobile device and is calibratable with respect to a reference location. The wireless communication unit is adapted to send position information to the medical system. Said position information is based on measurements of the at least one position sensor.

The mobile device further comprises mounting elements for attaching the mobile device to a defined location of an operating table. Such a defined location is, for example, a rail of the operating table. The mounting elements are, in particular, adapted to releasably mount a housing of the mobile device to the operating table.

The medical system may, in general, be any medical system that is adapted to be used with an operating table. In particular, the medical system may be mobile such that it can be moved to an operating table. Additionally, or alternatively, the operating table may be mobile, such that it can be moved to the medical system. In particular, the medical system comprises a mobile imaging system such as an X-ray system. In order to allow a relative motion of the medical system and the operating table, the medical system may be realized as a C-arm device.

The medical system comprises a wireless communication base unit. Said wireless communication base unit is adapted to receive position information sent by the wireless communication unit of the mobile device. The wireless communication base unit may be a USB enabled transceiver wireless device such as a USB Bluetooth transceiver module. The wireless communication base unit may be, for example, installed on a stand of the medical system. Also, the wireless communication base unit may be integrated with system software of the medical system.

The medical system further comprises a computing unit. Said computing unit may be a separate, dedicated computing unit but may also be an existing computing unit of the medical system equipped with additional software that enables it to perform the steps outlined below.

The medical system further comprises the reference location arranged to mount the mobile device. In particular, the reference location is on the mobile imaging system. Mounting the mobile device to the medical system may be performed using the mounting elements or by placing the mobile device on a marked spot on the medical system. At least for some time during the operation of the medical setup, the mobile device is to be placed at the reference location. As a simple example, the reference location may be a marked spot, e.g., on a stand of the medical system.

The computing unit is configured to calibrate an operating table with respect to the medical system, in particular with respect to the mobile imaging system. Said calibration is performed once the mobile device is mounted to the operating table. The calibration may include moving the mobile device from the reference location of the medical system to the defined location of the operating table. The at least one position sensor of the mobile device may measure the change in position from the reference location to the defined location such that the position of the defined location with respect to the reference location, and hence the position of the operating table with respect to the medical system, is known.

The computing unit is also configured to store the defined location as a known location in a coordinate system of the medical system, in particular of the mobile imaging system.

The computing unit is further configured to receive position information related to the measurements from the at least one position sensor via the wireless communication base unit. In particular, said measurements from the at least one position sensor are not only received during calibration of the operating table but also during subsequent operation of the medical system, in particular during a medical procedure. Hence, the position of the operating table with respect to the medical system may be known during the medical procedure.

With said medical setup, a variety of operating tables may be chosen. The medical system knows the position of the operating table, which may be used for a better control of the medical system, e.g., for placing a patient in an Isocenter of an X-ray system, which helps improving 3D image quality. The knowledge of the position of the operating table with respect to the medical system also allows a more precise determination of a patient dose. The knowledge of the position of the operating table with respect to the medical system may further allow a more precise positioning of the medical system, e.g., lowering a height of a C-arm and/or bringing a detector closer to a patient, hence reducing scatter radiation. The knowledge of the position of the operating table with respect to the medical system further helps preventing collisions between the medical system and the operating table, when one or both of them are moved.

According to an embodiment, the computing unit is further configured to generate control instructions to control the medical system and/or the operating table based on the received position information. As an example, for CT scans, the X-ray source and sensors have to be moved with respect to the patient, hence with respect to the operating table. This may be performed by moving the X-ray source and sensors, by moving the operating table or by both moving the X-ray source and sensors and the operating table. In order to determine the required movements of the X-ray source and sensors and/or the operating table, the received position information is used and the control commands comprise the determined movements.

According to an embodiment, the medical system further comprises a user interface unit. Said user interface unit may be a dedicated user interface unit or a standard user interface unit with added functionality.

The calibration of the operating table is performed using the user interface unit. This may comprise a step of asking a user, by the user interface unit, to place the mobile unit at the reference location and/or to confirm that the mobile unit is at the reference location. The calibration of the operating table may further comprise asking the user, by the user interface unit, to move the mobile device from the reference location to the defined location and to confirm that the mobile device has been placed at the defined location. Then, as explained above, position information is received from the at least one position sensor. Using this position information, the location of the operating table with respect to the medical system is determined.

According to an embodiment, the medical system further comprises a base station for the mobile device, wherein the reference location of the mobile device is on the base station. In particular, the base station may have a recess that corresponds to an outer shape of a part of the mobile device, such that the mobile device fits into the base station. Hence, the reference location is more well-defined and the subsequent measurements of the relative positions of the operating table may be improved.

The base station may further comprise a power charging base port. Said power charging base port is adapted to charge batteries of the mobile device. The charging may be performed, e.g., by wired charging or by inductive charging. Hence, while the mobile device is in the reference location, its batteries are also charged, such that it is ready for operation when needed.

In another aspect of the present invention, a system for calibrating an operating table in a medical environment is provided. In particular, the medical environment may comprise a medical system such as a medical imaging system and the medical system and/or the operating table are mobile.

The system comprises a mobile device, comprising a housing, mounting elements, at least one position sensor and a wireless communication unit. Preferably, the housing encases the position sensor(s) and the wireless communication unit.

The mounting elements are adapted to releasably mount the housing to the operating table. In particular, they are adapted to mount the housing to a defined location of the operating table, such as a rail of the operating table.

The position sensor(s) are adapted to measure a position of the mobile device in a fixed coordinate system. Said fixed coordinate system is, in particular, a coordinate system attached to the medical system.

The wireless communication unit is adapted to transmit position information measured by the position sensor(s). A wireless standard, such as Bluetooth, that has a range of up to a few meters, may suffice.

The system further comprises a user interface unit. Said user interface unit may be an existing user interface unit of a medical system or may be a dedicated user interface unit for the system.

The user interface unit is arranged to wirelessly receive position information from the mobile device. For this, the user interface unit comprises or is connected to a wireless communication base unit that shares at least one standard and/or frequency band with the wireless communication unit of the mobile device.

The user interface is further arranged to register the operating table to the fixed coordinate system. To do so, the user interface registers the position of the mobile device to the fixed coordinate system once mounted on the operating table. This may, for example, be achieved by measuring the change in position of the mobile device when it is moved from a reference location on the medical system and/or user interface unit to the defined location on the operating table.

Then, the position of the mobile device to a geometrical predetermined reference of the operating table is registered. Said geometrical predetermined reference may be the defined location, a top flat surface of the operating table or an Isocenter of the medical system. The registration data is then stored in a memory. Said memory may be a memory of the user interface unit and/or a memory of the medical system.

Then, the operating table is calibrated by inputting reference values corresponding to positions in the use of the operating table, such as maximum and minimum position values of the operating table along directions of the fixed coordinate system. To obtain said maximum and minimum position values, the user interface unit may ask a user to move the operating table to the respective maximum and minimum position values, e.g., in the three Cartesian coordinates. These calibration values are then stored in the memory.

Alternatively, or additionally, a previously calibrated operating table stored in the memory is read and selected and the corresponding calibration values are uploaded from the memory. This saves time in calibrating an operating table that has been used before.

With said system for calibrating an operating table, a variety of operating tables may be chosen. The system knows the position of the operating table, which may be used for a better control of the medical system, e.g., for placing a patient in an Isocenter of an X-ray system, which helps improving 3D image quality. The knowledge of the position of the operating table with respect to the medical system also allows a more precise determination of a patient dose. The knowledge of the position of the operating table with respect to the medical system may further allow a more precise positioning of the medical system, e.g., lowering a height of a C-arm and/or bringing a detector closer to a patient, hence reducing scatter radiation. The knowledge of the position of the operating table with respect to the medical system further helps preventing collisions between the medical system and the operating table, when one or both of them are moved.

An initial position of the operating table when a medical procedure starts may be obtained using the mobile device and stored in a memory. That way, changes of the position of the operating table compared to said initial position may be used during the medical procedure. In this context, the medical procedure may be a surgical procedure and/or a medical imaging procedure.

In yet another aspect of the present invention, a mobile device for calibrating an operating room table is provided. The mobile device comprises at least one position sensor. Said position sensor is adapted to measure a position of the mobile device in a fixed coordinate system.

The mobile device further comprises a controlling unit. Said controlling unit may, in particular, be a micro-controller with embedded software. The controlling unit is configured to receive measurements from the at least one position sensor and broadcast said measurements and/or position information related to said measurements via a wireless communication unit of the mobile device. The wireless communication unit may be, for example, a Bluetooth low energy transceiver.

The mobile device further comprises a battery for providing the at least one position sensor, the controlling unit and the wireless communication unit with electric energy. In particular, the battery may be a rechargeable battery. In this case, the mobile device also comprises charging means, e.g., a charging adaptor with a port to connect a charger and/or a wireless charging unit.

The mobile device further comprises mounting elements for attaching the mobile device to a defined location of an operating table. In particular, this attachment is a releasable attachment, e.g., with a mounting bracket. Said release of the mobile device may be further eased by a knob or the like. The defined location is, in particular, a rail of the operating table.

The mobile device may further comprise a housing that encases, in particular, the at least one position sensor, the controlling unit, the wireless communication unit and the battery.

With said mobile device, a calibration of an operating table may be performed. The advantages of such a calibration have been described in detail above.

According to an embodiment, the at least one position sensor is an inertial measurement unit comprising an accelerometer and a gyroscope, i.e., a 6 degrees-of-freedom sensor. By continuously measuring the linear and rotational acceleration, the inertial measurement unit may provide a three-dimensional change in position, in particular a distance moved and an orientation. In particular, the at least one position sensor may further comprise a magnetometer, making it a 9 degrees-of-freedom sensor. The use of a magnetometer improves the accuracy of the measurements of the accelerometer and the gyroscope, in particular by reducing or eliminating a gyro drift or a long term drift.

In yet another aspect of the present invention, a method for operating medical equipment is provided. The medical equipment comprises a medical setup according to the above description and an operating table. At least one of the medical system and the operating table is mobile such that the medical system and the operating table can be brought into an operating location relative to one another.

According to the method, the mobile device is moved, by a user, from the reference location on the medical system to the defined location of the operating table. Since the position and orientation of the mobile device are known at the beginning of this move, the position of the mobile device at the end of the move, relative to the medical system, is also known. And since the mobile device is placed at a defined location of the operating table, this position implies a position of the operating table.

As a next step, the operating table is initialized, i.e., the operating table is registered with the medical setup.

Further, in particular during a medical procedure such as a surgical procedure or a medical imaging procedure, measurements are performed by the at least one position sensor and position information related to the measurements is broadcast by the wireless communication unit of the mobile device and received by the wireless communication base unit of the medical setup.

According to an embodiment, control instructions to control the medical system and/or the operating table are generated by the computing unit based on said received position information. Said control instructions may, in particular, be instructions to move parts of the medical system and/or the operating table. Since the computing unit is a part of the medical setup, the control instructions to control parts of the medical system may be directly executed by the medical system. Regarding control instructions to move the operating table, a connection between the medical setup and the operating table may be used to execute these instructions, or the instructions may be displayed on a display, e.g., of the user interface unit, and a user may operate the operating table such that the desired movements are performed. In the latter case, the measurements taken by the mobile device may then be used to confirm that the correct movements have been performed.

According to an embodiment, initializing the operating table comprises moving the operating table to a default position. This may be performed before or after the mobile device has been moved from the reference location to the defined location. Based on this default position, a range of movements of the operating table is or will be known. If the operating table has no inherent default position, a default position may be defined, e.g., as position that is the most left, most to the front and lowest. This definition may also be indicated on the user interface unit.

According to an embodiment, initializing the operating table comprises obtaining an operating table moving range calibration. This is in particular needed when a new table is registered with the medical setup. For obtaining the moving range, the position of the mobile device is registered, in particular in the default position, i.e., the position of the mobile device with respect to the medical system is saved. Then, for at least one direction out of three directions in which the operating table may be moved, the operating table is moved to one end point in said direction. There, the position of the mobile device is registered, i.e., the position with respect to the medical system is saved. Then, the operating table is moved to another end point in said direction, the other end point being the opposite end to the one end point. Also at the other end point, the position of the mobile device is registered. Then, the moving range of the operating table in said direction is known. If the operating table can be moved in more than one direction, the above steps are repeated for the other directions. In particular, the movements of the operating table are controlled by a user based on instructions given on a display of the user interface unit. In this context, the three directions may be an X-direction, a Y-direction and a Z-direction in a coordinate system of the operating table.

According to an embodiment, the above moving range calibration may also performed for rotations around at least one axis out of three axes, wherein the three axes are, in particular, along an X-direction, a Y-direction and a Z-direction in a coordinate system of the operating table. For this, the position of the mobile device is registered. Then, the operating table is rotated around the axis to one end point and the position of the mobile device is registered. Then, the operating table is rotated around the axis to another end point, the other end point being opposite to the one end point. The position of the mobile device is also registered in this position. With this, also the range of rotations of the operating table is known.

According to an embodiment, obtaining the operating table moving range calibration further comprises moving the mobile device from the defined location to a predetermined location and determining the offset between the defined location and the predetermined location. In particular, the mobile device is then moved back to the defined location. In an alternative embodiment, the mobile device may be moved from the reference location to the predetermined location and then to the defined location, which saves one step in initializing the operating table. The predetermined location is, in particular, a position on top of the operating table, more particularly a center position of the top of the operating table.

According to an embodiment, obtaining the operating table moving range calibration further comprises storing a height value of the mobile device as reference value. Since the height above the floor of the reference location is known, said height of the mobile device in the defined location can be easily determined using the relative location of the mobile device with respect to the medical setup. Once the moving range calibrations have been performed, the calibration values may be saved for later use, in particular using an identifier such as a brand name, model name, serial number or color of the operating table, for the operating table.

According to an embodiment, obtaining the operating table moving range calibration comprises selecting a previously calibrated operating table stored in a memory and reading and uploading the corresponding calibration values from the memory. In particular, the operating table may be identified by the identifier. Said selection may be performed, e.g., with a drop down menu of the user interface unit. The memory may be a memory of the computing unit, i.e., associated with the medical system, but may also be a memory of the mobile device. By selecting a previously calibrated operating table, only the position of the mobile device at the defined location relative to the medical system has to be determined, and the determination of the moving ranges can be skipped, hence saving a lot of time.

According to an embodiment, initializing the operating table comprises selecting, by the user, an operating table location where the mobile device is mounted. In particular, it may be indicated by the user on which rail of the operating table, e.g., on the left top, left bottom, right top or right bottom, the mobile device has been mounted. It may be further indicated by the user on which side of the operating table, i.e., the left side or the right side, the medical system is located.

According to an embodiment, initializing the operating table comprises storing an initial position of the operating table before or when a medical procedure starts. In this context, a medical procedure may be a surgical procedure or a medical imaging procedure. This position also initializes the patient position for said medical procedure, such that position changes during the medical procedure can always be related to the stored initial position.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic cross section through an embodiment of a mobile device;
Fig. 2 shows a schematic side view of an embodiment of a medical setup;
Figs. 3a and 3b show an embodiment of a method for operating the medical setup;
Fig. 4 shows another embodiment of a method for operating the medical setup; and
Fig. 5 shows a schematic side view of another embodiment of a medical setup.

### DETAILED DESCRIPTION OF EMBODIMENTS

Like numbered elements in these figures are either equivalent elements or perform the same function. Elements which have been discussed previously will not necessarily be discussed in later figures if the function is equivalent.

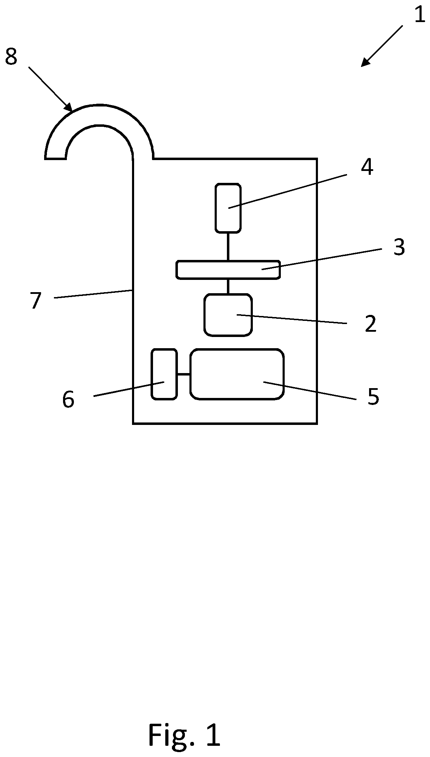

Fig. 1 shows a schematic cross-section through an embodiment of a mobile device 1. The mobile device 1 comprises at least one position sensor 2. Said position sensor 2 is adapted to measure a relative position of the mobile device 1 to a medical system. For example, the at least one position sensor 2 may be an inertial measurement unit comprising an accelerometer and a gyroscope, in particular a three-axes accelerometer and a three-axes gyroscope. With said accelerometer and gyroscope, a movement - both translational and rotational - of the mobile device 1 may be determined and, starting from a known starting position, the final position may be determined relative to said known starting position. To further improve the accuracy of the position determination, the at least one position sensor 2 may further comprise a magnetometer, which may reduce or eliminate a gyro drift or a long term drift.

The mobile device 1 further comprises a controlling unit 3, e.g., a microcontroller. Said controlling unit 3 receives sensor measurements and/or sensor signals from the at least one position sensor 2. The controlling unit 3 may process said sensor signals, e.g., integrate the accelerations to compute a change in position.

The mobile device 1 also comprises a wireless communication unit 4, which receives information from the controlling unit 3, e.g., sensor measurements or processed sensor measurements such as position changes, and broadcasts said information, e.g., to a medical system.

Electric power may be supplied to the mobile device 1 by a battery 5. Said battery 5 is connected to the at least one position sensor 2, the controlling unit 3 and the wireless communication unit 4, however, said connections are not shown in the figure for clearness. The battery 5 is further connected to a charging unit 6, which may be a wired or a wireless (e.g., inductive) charging unit.

The position sensor 2, the controlling unit 3, the wireless communication unit 4, the battery 5 and the charging unit 6 are all enclosed in a housing 7.

Mounting elements 8, here depicted as hooks, are attached to the housing 7 or are a part of the housing 7. Said mounting elements 8 are adapted to attach the mobile device 1 to a defined location of an operating table, such as a rail. There may be further means for securing the mounting elements 8 to the defined location or release means such as a knob to release the mobile device 1 from the defined location of the operating table.

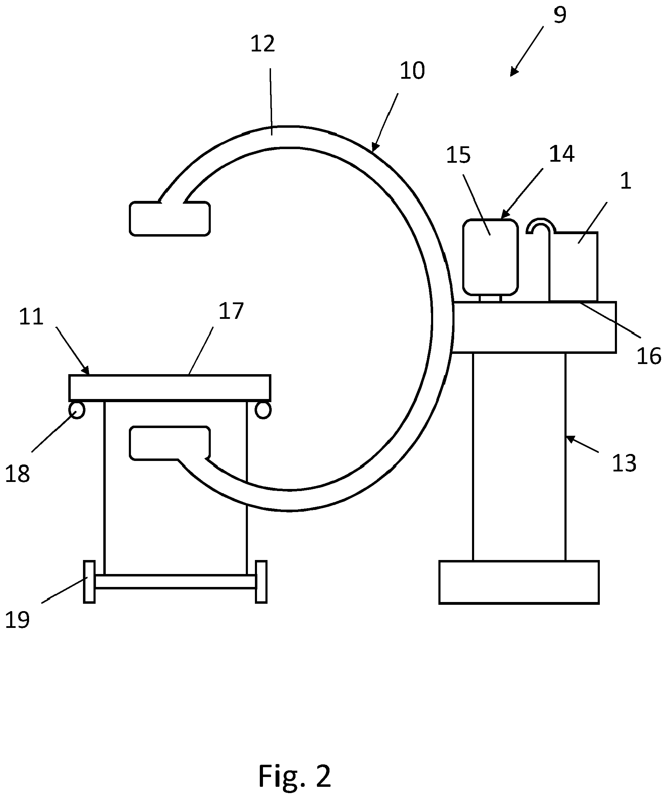

Fig. 2 shows a medical setup 9 comprising a medical system 10 and a mobile device 1 as well as an operating table 11.

The medical system 10 is shown as a C-arm X-ray system, with a C-arm 12 and a stand 13. However, a plurality of other medical systems 10 may be used, in particular medical system 10 that need to know a relative position of the operating table 11 to the medical system 10. The medical system 10 further comprises a user interface unit 14 with a display 15. The mobile device 1 is placed at a reference location 16 of the medical system 10, which may indicated, e.g., by a drawn circumference of the mobile device 1 on the stand 13 of the medical system 10.

The operating table 11 has a top 17 of the operating table 11 and comprises, among others, rails 18. Further, the operating table 11 is shown as a mobile operating table 11 with wheels 19. However, the invention also applies to a stationary operating table 11 and a mobile medical system 10 as well as to a mobile operating table 11 and a mobile medical system 10.

Before a medical procedure, such as a surgical procedure or a medical imaging procedure, starts, the operating table 11 may be initialized as follows.

The user interface unit 14 may provide several options to a user, e.g., to add a table, to select a previously configured table, and to initialize a table.

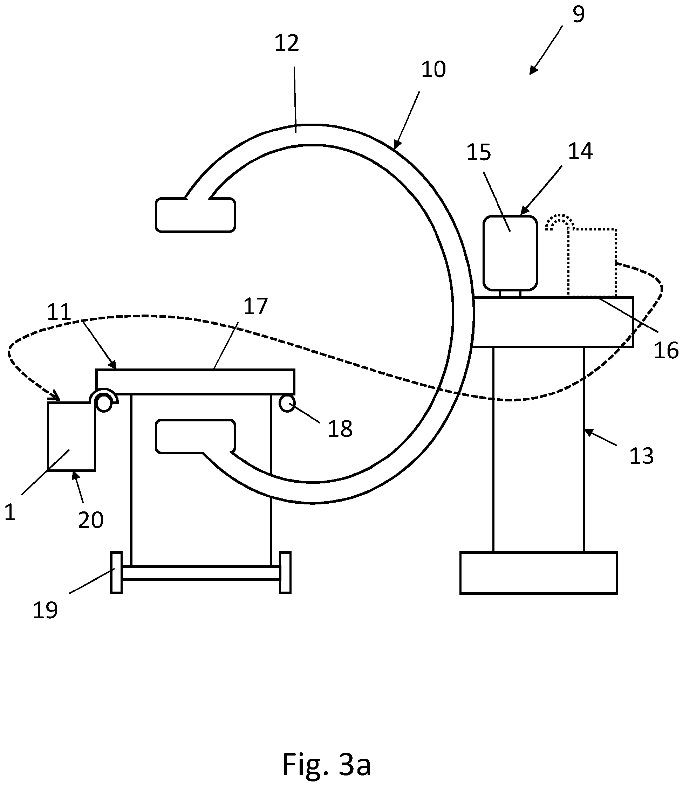

When the user selects to add a table, he/she will be asked to put the mobile device 1 on the reference location 16 or to confirm that the mobile device 1 already is on the reference location 16. The user is then asked to move the mobile device 1 to defined location 20 on the operating table 11, e.g., to mount it to one of the rails 18, as shown in Fig. 3a. During this movement, the mobile device 1 measures the change in its position, such that the relative position of the defined location 20 to the reference location 16 is known. Then, the user may select on the user interface unit 14 the orientation of the mobile device 1, i.e., on which rail 18 of the operating table 11 it has been mounted. Also, the user may select on which side of the operating table 11 the medical system 10 is located.

Before or after moving the mobile device 1 to the defined location 20, the user may be asked to put the operating table 11 in a default position, e.g., a default position provided by the table or a pre-defined default position such as the minimum height, towards a patient's legs and to the right most of the patient.

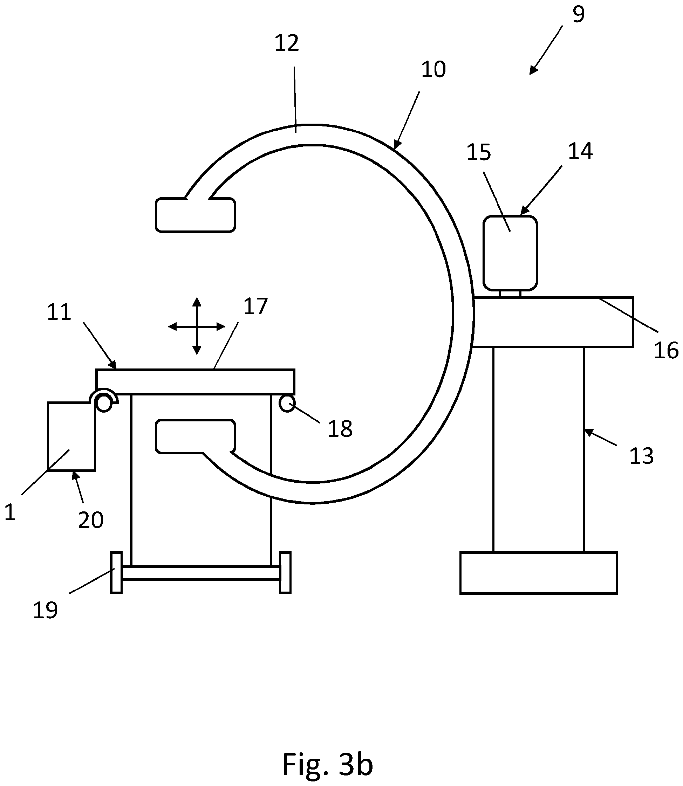

After the mobile device has been moved to the defined location 20 and after the operating table 11 has been put in its default position, the user is asked to move the operating table 11 to its maximum and minimum positions in all three directions as indicated by the arrows in Fig. 3b and, if available, rotate the operating table 11 to its maximum and minimum positions around all available axes. These motions are recorded by the position sensor(s) 2 of the mobile device 1 such that the extreme positions of the operating table 11 are known to the medical setup 9. This procedure is finished by selecting initialize a table by the user. In this step, also a name or label may be assigned to the table such that it can be chosen at a later date, which saves the steps of finding the maximum movement ranges.

When a previously configured operating table 11 is selected, e.g., from a drop-down menu, the user only has to move the operating table 11 to its default position, and move the mobile device 1 from the reference location 16 to the defined location 20.

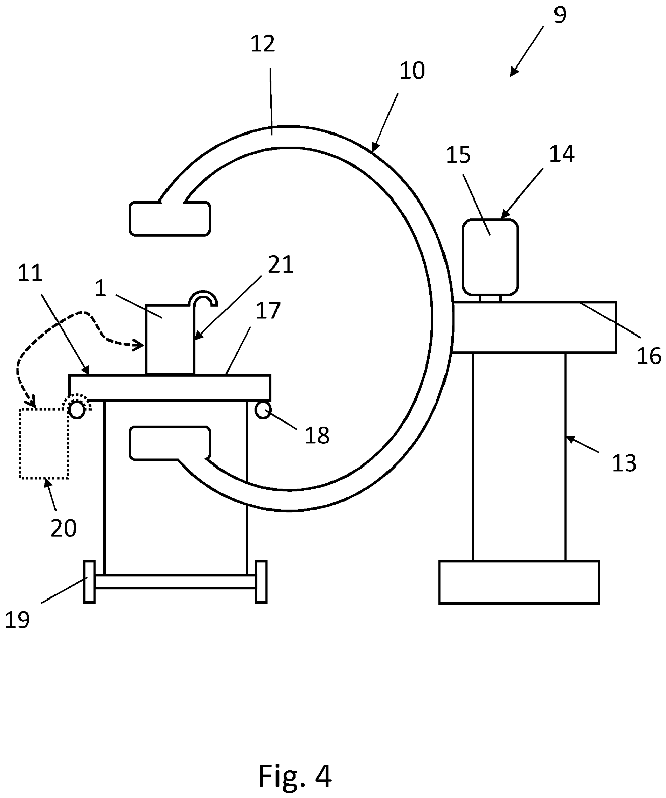

Another embodiment of a method for operating the medical setup 9 comprises an additional step shown in Fig. 4. Here, the mobile device 1 is moved from the defined location 20 to a predetermined location 21, in particular on top 17 of the operating table 11, where, e.g., the patient is or will be located. Once the relative position of the predetermined location 21 with respect to the defined location 20 - and hence with respect to the reference location 16 - has been determined, the mobile device 1 is moved back to the defined location 20. Knowing the predetermined location 21 on top 17 of the operating table 11 will allow for an even more precise relative placement of the operating table 11 with respect to the medical system 10.

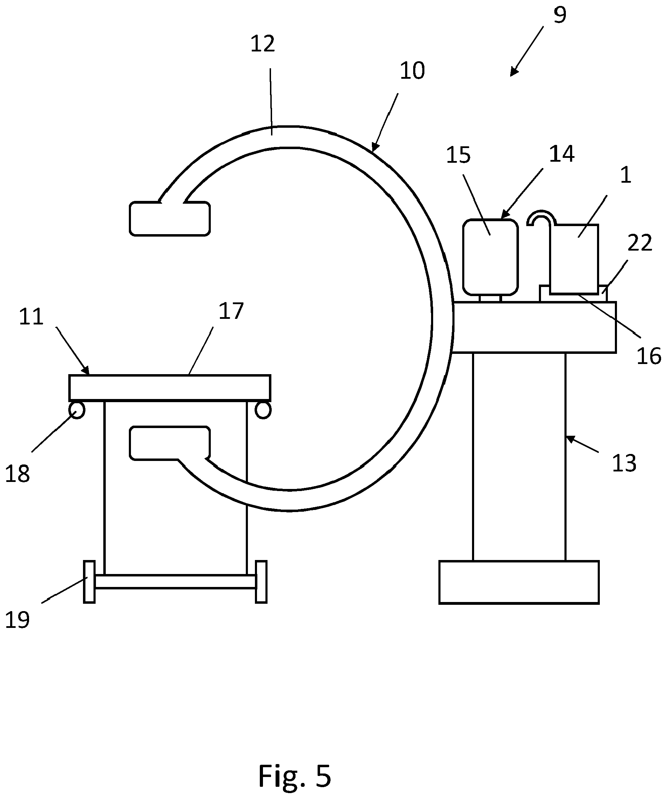

In yet another embodiment, shown in Fig. 5, a base station 22 is arranged on the stand 13 of the medical system 10. Said base station 22 is adapted to receive the mobile device 1 and provides the reference location 16 for the mobile device 1. Further, the base station 22 may comprise a power charging base port, to charge the battery 5 of the mobile device 1.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: mobile device
- 2: position sensor
- 3: controlling unit
- 4: wireless communication unit
- 5: battery
- 6: charging unit
- 7: housing
- 8: mounting element
- 9: medical setup
- 10: medical system
- 11: operating table
- 12: C-arm
- 13: stand
- 14: user interface unit
- 15: display
- 16: reference location
- 17: top
- 18: rail
- 19: wheel
- 20: defined location
- 21: predetermined location
- 22: base station

## Claims

1. Medical setup (9), comprising:
a mobile device (1), comprising:
at least one position sensor (2) calibratable with respect to a reference location;
a wireless communication unit (4); and
mounting elements (8) for attaching the mobile device (1) to a defined location (20) of an operating table (11); and
a medical system (10), in particular comprising a mobile imaging system, comprising
a wireless communication base unit;
a computing unit; and
the reference location (16), in particular on the mobile imaging system, arranged to mount the mobile device (1);
wherein the computing unit is configured to
calibrate an operating table (11) with respect to the medical system (10), in particular with respect to the mobile imaging system, once the mobile device (1) is mounted to the operating table (11);
store the defined location (20) as a known location in a coordinate system of the medical system (10), in particular of the mobile imaging system; and
receive position information related to the measurements from the at least one position sensor (2) via the wireless communication base unit.

2. Medical setup (9) according to claim 1, wherein the computing unit is further configured to:
generate control instructions to control the medical system (10) and/or the operating table (11) based on the received position information.

3. Medical setup (9) according to claim 1 or 2, wherein
the medical system (10) further comprises a user interface unit (14); and
the calibration of the operating table (11) is performed using the user interface unit (14), in particular, including receiving position information from the at least one position sensor (2) after the mobile device (1) has been moved by a user from the reference location (16) to the defined location (20).

4. Medical setup (9) according to any of claims 1 to 3, wherein the medical system (10) further comprises a base station (22) for the mobile device (1), wherein the reference location (16) of the mobile device (1) is on the base station (22), and wherein, in particular, the base station (22) comprises a power charging base port.

5. System for calibrating an operating table (11) in a medical environment, comprising:
a mobile device (1), comprising:
a housing (7);
mounting elements (8) to releasably mount the housing (7) to the operating table (11);
at least one position sensor (2) in the housing (7) to measure a position of the mobile device (1) in a fixed coordinate system; and
a wireless communication unit (4) to transmit position information measured by the position sensor(s) (2); and
a user interface unit (14) arranged to:
wirelessly receive position information from the mobile device (1);
register the operating table (11) to the fixed coordinate system, by
registering the position of the mobile device (1) to the fixed coordinate system once mounted on the operating table (11);
registering the position of the mobile device (1) to a geometrical predetermined reference of the operating table (11); and
storing in a memory this registration data; and
calibrate the operating table (11) comprising input reference values corresponding to positions in the use of the operating table (11), and store these calibration values in the memory; and/or
read and select a previously calibrated operating table (11) stored in the memory, and upload the corresponding calibration values from the memory.

6. Mobile device (1) for calibrating an operating room table, comprising:
at least one position sensor (2);
a controlling unit (3);
a wireless communication unit (4);
a battery (5); and
mounting elements (8) for attaching the mobile device (1) to a defined location (20) of an operating table (11),
wherein the controlling unit (3) is configured to receive measurements from the at least one position sensor (2) and broadcast position information related to the measurements via the wireless communication unit (4).

7. Mobile device (1) according to claim 6, wherein the at least one position sensor (2) is an inertial measurement unit comprising an accelerometer and a gyroscope and, in particular, a magnetometer.

8. Method for operating medical equipment, wherein
the medical equipment comprises
a medical setup (9) according to any of claims 1 to 4; and
an operating table (11); and
the method comprises
moving, by a user, the mobile device (1) from the reference location (16) to the defined location (20) of the operating table (11);
initializing the operating table (11);
performing measurements by the at least one position sensor (2); broadcasting position information related to the measurements by the wireless communication unit (4); and
receiving the position information by the wireless communication base unit.

9. Method according to claim 8, wherein the method further comprises:
generating control instructions to control the medical system (10) and/or the operating table (11) based on the received position information by the computing unit.

10. Method according to any of claims 8, wherein initializing the operating table (11) comprises obtaining an operating table (11) moving range calibration, comprising:
registering the position of the mobile device (1); and
for at least one direction out of three directions:
moving the operating table (11) to one end point in the direction;
registering the position of the mobile device (1);
moving the operating table (11) to another end point in the direction; and
registering the position of the mobile device (1),
wherein the three directions are, in particular, an X-direction, a Y-direction and a Z-direction in a coordinate system of the operating table (11).

11. Method according to claim 10, wherein obtaining the operating table (11) moving range calibration further comprises
registering the position of the mobile device (1); and
for at least one axis out of three axes:
rotating the operating table (11) to one end point around the axis;
registering the position of the mobile device (1);
rotating the operating table (11) to another end point around the axis; and
registering the position of the mobile device (1),
wherein the three axes are, in particular, along an X-direction, a Y-direction and a Z-direction in a coordinate system of the operating table (11).

12. Method according to claim 10 or 11, wherein obtaining the operating table (11) moving range calibration further comprises moving the mobile device (1) from the defined location (20) to a predetermined location (21), in particular on top (17) of the operating table (11), and determining the offset between the defined location (20) and the predetermined location (21).

13. Method according to any of claims 10 to 12, wherein obtaining the operating table (11) moving range calibration comprises storing a height value of the mobile device (1) as reference value.

14. Method according to any of claims 10 to 13, wherein obtaining the operating table (11) moving range calibration comprises selecting a previously calibrated operating table (11) stored in a memory, reading and uploading the corresponding calibration values from the memory.

15. Method according to any of claims 8 to 14, wherein initializing the operating table (11) comprises selecting, by the user, an operating table (11) location where the mobile device (1) is mounted.
